# EUROPEAN PATENT SPECIFICATION

(11) **EP 2 097 435 B1**
(45) Date of publication and mention of the grant of the patent: **02.07.2014**
(21) Application number: 07839791.6
(22) Date of filing: 26.10.2007
(51) Int. Cl.: C07H 21/02, A01N 43/04

(54) **THERAPEUTIC TREATMENTS BASED ON ADMINISTRATION OF SMALL RNA FRAGMENTS**
AUF DER VERABREICHUNG KLEINER RNA-FRAGMENTE BASIERENDE THERAPEUTISCHE BEHANDLUNGEN
TRAITEMENTS THÉRAPEUTIQUES BASÉS SUR L'ADMINISTRATION DE PETITS FRAGMENTS D'ARN

(30) Priority: 27.10.2006 US 854841 P
(43) Date of publication of application: 09.09.2009
(73) Proprietor: Molecular International Research, Inc., New York NY 10022 (US)
(72) Inventor: BELJANSKI, Sylvie, New York, NY 10128 (US); HALL, John, New York, NY 10022 (US)
(74) Representative: Wright, Simon Mark
(86) International application number: PCT/US2007/022653
(87) International publication number: WO 2008/057256

(56) References cited:
- US-A- 4 190 649
- US-A- 4 335 239
- BELJANSKI M ET AL: "LEUKOCYTE RECOVERY WITH SHORT CHAIN RNA FRAGMENTS IN CYCLO PHOSPHAMIDE TREATED RABBITS", CANCER TREATMENT REPORTS, vol. 67, no. 7-8, 1983, pages 611-620, XP009154362, ISSN: 0361-5960
- WANG JIANPING ET AL: "Effect of nucleosides and a nucleotide mixture on proliferation of human gastric cancer cells (KATO III)", KOBE JOURNAL OF MEDICAL SCIENCES, vol. 40, no. 2, 1994, pages 65-75, XP009154365, ISSN: 0023-2513
- LEVIN ROBERT D ET AL: "Dose escalation study of an anti-thrombocytopenic agent in patients with chemotherapy induced thrombocytopenia.", BMC CANCER, vol. 10, 565, 2010, pages 1-8, XP002664445, ISSN: 1471-2407
- T KOJIMA ET AL: "The effects of multiple combination chemotherapy with vincristine, cyclophosphamide (Endoxan), methotrexate, 5-fluorouracil, adriamycin and prednisolone (VEMFAH) for advanced breast cancer", CANCER CHEMOTHERAPY AND PHARMACOLOGY, vol. 15, no. 3, 1 January 1985 (1985-01-01), pages 268-271, XP055053433, ISSN: 0344-5704

## Description

### FIELD OF THE INVENTION

This invention generally relates to therapeutic treatments that prevent or ameliorate thrombocytopenia. More specifically, the invention provides an improved chemotherapeutic preparation for use in a method to prevent or ameliorate bone marrow suppression, and specifically thrombocytopenia, induced by anti-cancer drugs. This invention also provides a therapeutic preparation for use in the treatment for thrombocytopenia associated with immune disorders known as Immune Thrombocytopenic Purpura. More particularly, the therapeutic preparations provided by the present invention comprise small RNA fragments.

### BACKGROUND OF THE INVENTION

Chemotherapy is the foremost treatment for the majority of cancers. While oncologists have improved the standard of therapy with various drug combinations and with newer drugs having reduced side effects, the fact remains that chemotherapy is often associated with complications that are at best unpleasant and at worst devastating. In cases of advanced cancers, metastases, or tumors that are resistant to treatment, chemotherapy may be used in especially aggressive forms that commonly lead to bone marrow suppression, reduced immunity, low platelet counts and consequent death of the patient. A number of agents that help support white blood cell counts have been used in conjunction with such therapies, but these agents are not always effective and they do not, in any event, stimulate the production of platelets. The critical drop in platelet levels induced by chemotherapy, a condition called thrombocytopenia, is frequently the main factor in the demise of the patient who, because their blood can no longer clot effectively, is susceptible to uncontrolled internal as well as external bleeding.

Thrombocytopenia induced by chemotherapy is also a major obstacle for the clinical oncologist whose goal is to shrink the patient's tumor and slow the advance of cancer. Because a patient's platelet count is so critical, this number is carefully monitored throughout the course of chemotherapeutic treatment, and when the number drops below a safe threshold, the doses are reduced or the treatment is suspended until the platelet count has a chance to rebound. In patients who have undergone previous rounds of chemotherapy and whose bone marrow has suffered permanent damage, the recovery of platelet count may take considerable time and may not occur at all. As a result, the loss of platelets that is induced by anti-cancer drugs is currently the rate-limiting step in chemotherapy. As thrombocytopenia is reached (Grade 3, 10,000-50,000 platelets per milliliter of blood; Grade 4, <10,000 platelets per milliliter of blood), the risks of lethal hemorrhage are acute and many cancer patients have succumbed to uncontrolled bleeding. In addition, for a patient in Grade 4 thrombocytopenia, the treatment must be reduced or suspended until platelet levels increase above threshold.

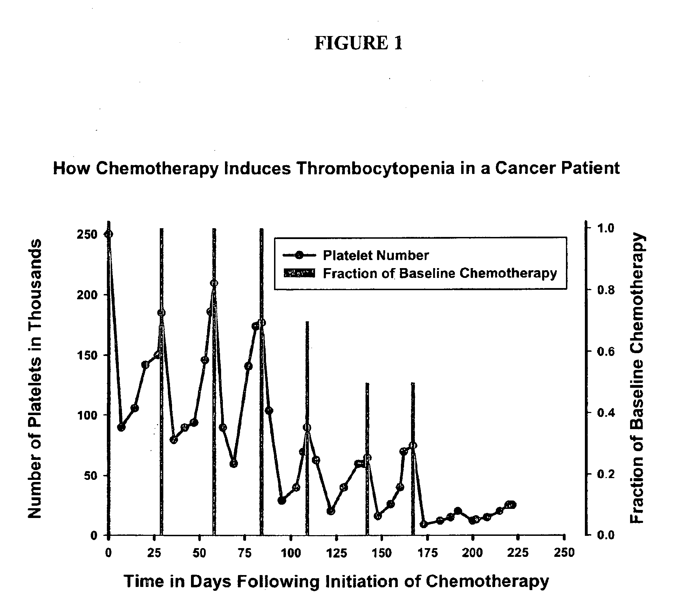

Chemotherapy is normally given periodically in multiple cycles continuing for many weeks. The spacing between cycles enables most patients at the beginning of treatment to renew their population of platelets - usually in a matter of days - before they undergo the next cycle of chemotherapy. As the treatment progresses the collapse in platelet number following chemotherapy (nadir) becomes more severe and the process of renewal takes longer. Typically, the patient's capacity to restore safe platelet counts is progressively diminished as seen in **Figure 1**. It is common for the drop in platelet counts and/or the subsequent delay or failure of proliferation to force the oncologist to suspend treatment. This is when thrombocytopenia becomes rate limiting in cancer chemotherapy and this condition is the foremost reason why treatment is derailed, which means that the goal of shrinking or destroying the patient's tumor is not accomplished.

This fundamental problem exists throughout all stages of chemotherapy. Cancer therapy may entail multiple cycles of chemotherapy and many oncologists consider that thrombocytopenia has a critical effect in the early stages of treatment as well as at later stages. In the early stages oncologists are often selecting the optimal mix of drugs that induces the best response in the patient. This critical phase of matching the treatment to the individual's cancer to obtain the best therapeutic effect is interrupted when treatment is suspended because of thrombocytopenia. The tumor gets a respite just when the treatment should become optimal and the cancer may advance possibly metastasizing and becoming more refractory to further treatment. Successful therapy for these patients requires a new regimen that sustains platelet counts.

As noted above, in patients with later stage cancers who have undergone previous cycles of chemotherapy, the repopulation of white blood cells and especially platelets is compromised by permanent damage to the stem cells in the bone marrow. Successful treatment of these patients requires a new regimen that protects bone marrow stem cells and can stimulate the production of platelets even in cases of bone marrow damage. The present invention provides a chemotherapeutic regimen that fulfills both of these critical needs and thus has the potential to revolutionize cancer chemotherapy.

Thrombocytopenia is also a significant problem for individuals suffering from defects in platelet production and processing associated with a disease known as Immune Thrombocytopenic Purpura (ITP). Individuals-both children and adults may be afflicted-who suffer from this disease fail to maintain normal platelet counts due to the accelerated destruction of platelets and deficient platelet production. In ITP, anti-platelet antibodies are generated as part of an auto-immune response that be may influenced by genetic factors. Although the detailed etiology of ITP is still unknown, it is the presence of these antibodies that causes critical loss of platelets and that also appears to be a factor in reducing platelet production in the bone marrow. As a result, these individuals confront the same clinical symptoms as patients suffering from bone marrow suppression induced by chemotherapy, including enhanced risk of internal and external bleeding and in extreme cases, life-threatening hemorrhage. It is estimated that approximately 200,000 individuals are diagnosed with this condition in the United States every year. Compared to the incidence thrombocytopenia found in patients undergoing myelosuppressive therapy, ITP is relatively rare and it is classified as an orphan disease. But despite different causes, the underlying pathology and the clinical symptoms of ITP and drug-induced thrombocytopenia are consistent and the present invention describes an agent that effectively stimulates platelet production and thus successfully treats thrombocytopenia in both patient populations.

### SUMMARY OF THE INVENTION

The present invention provides therapeutic preparations for use in methods of treatment that prevent or ameliorate thrombocytopenia, which comprise small RNA fragments. Thrombocytopenia suitable for treatment in accordance with the present invention includes thrombocytopenia induced by anti-cancer drugs in a chemotherapy regimen and congenital Immune Thrombocytopenic Purpura (ITP).

In one embodiment, the therapeutic preparation of the present invention is for administration to cancer patients undergoing a chemotherapeutic regimen to prevent or ameliorate bone marrow suppression, and specifically thrombocytopenia, induced by anti-cancer drugs.

This invention provides for the synchronization of the administration of anti-cancer drugs with the administration of a preparation of small RNA fragments. The preparation of small RNA fragments is believed to have protective effects on bone marrow through stimulating the proliferation of white blood cells and platelets. The protective effects on bone marrow enable patients to maintain their white blood cell counts and their platelet counts thus averting infection and bleeding. Oncologists prescribing a regimen in accordance with the present invention can now optimize chemotherapies that will be completed without interruption to attain the full benefit of the chemotherapy. Patients taking this regimen are protected from the effects of bone marrow suppression, and can complete therapy and live longer.

In another embodiment, the present invention provides a preparation comprising small RNA fragments for use in a method of treating ITP. The preparation of small RNA fragments stimulates the production of platelets thereby alleviating the symptoms associated with the disease. Individuals with ITP who are administered RNA fragments show significant improvements in their platelet counts, are no longer at risk for bleeding, and experience better quality of life.

Accordingly, the present invention provides a preparation of small RNA fragments and at least one anti-cancer compound for use in the treatment of a solid tumour in a human patient, wherein
said small RNA fragments are bacterial small RNA fragments composed of single chain polyribonucleotides having 10 to 80 ribonucleotide units, and an overall ratio of purine bases to pyrimidine bases [(G+A)/(C+U)] of between 1.0 and 2.5, and further wherein
said small RNA fragments are administered at a dose of at least 60 mg to a subject at least every other day to maintain the platelet level or permit the recovery of platelets to a normal level during and after the administration of said anti-cancer compound.

The invention also provides the use of small RNA fragments in the manufacture of a preparation for:
stimulating the proliferation and accelerating the regeneration of platelets in a cancer patient with a solid tumour undergoing chemotherapy with an anti-cancer drug; or
stimulating the proliferation and accelerating the regeneration of thrombocytes in a cancer patient with a solid tumour undergoing chemotherapy with an anti-cancer drug or;
stimulating the proliferation of white blood cells in a cancer patient with a solid tumour undergoing chemotherapy with an anti-cancer drug, wherein
said small RNA fragments are bacterial small RNA fragments composed of single chain polyribonucleotides having 10 to 80 ribonucleotide units, and an overall ratio of purine bases to pyrimidine bases [(G+A)/(C+U)] of between 1.0 and 2.5, and further wherein
said small RNA fragments are administered at a dose of at least 60 mg to a subject at least every other day to maintain the platelet level or permit the recovery of platelets to a normal level during and after the administration of said anti-cancer compound.

The invention further provides a preparation of small RNA fragments for use in the treatment of thrombocytopenia in a human patient suffering from Immune Thrombocytopenic Pupura, wherein
said small RNA fragments are bacterial small RNA fragments composed of single chain polyribonucleotides having 10 to 80 ribonucleotide units, and an overall ratio of purine bases to pyrimidine bases [(G+A)/(C+U)] of between 1.0 and 2.5, and further wherein
said small RNA fragments are administered at a dose of at least 20 mg/week and for a time sufficient to raise the platelet count.

The invention further provides the use of a preparation of small RNA fragments and at least one anti-cancer compound for the manufacture of a medicament for the treatment of a solid tumour in a human patient, wherein
said small RNA fragments are bacterial small RNA fragments composed of single chain polyribonucleotides having 10 to 80 ribonucleotide units, and an overall ratio of purine bases to pyrimidine bases [(G+A)/(C+U)] of between 1.0 and 2.5, and further wherein
said small RNA fragments are administered at a dose of at least 60 mg to a subject at least every other day to maintain the platelet level or permit the recovery of platelets to a normal level during and after the administration of said anti-cancer compound.

The invention also provides the use of a preparation of small RNA fragments for the manufacture of a medicament for the treatment of thrombocytopenia in a human patient suffering from Immune Thrombocytopenic Pupura wherein
said small RNA fragments are bacterial small RNA fragments composed of single chain polyribonucleotides having 10 to 80 ribonucleotide units, and an overall ratio of purine bases to pyrimidine bases [(G+A)/(C+U)] of between 1.0 and 2.5, and further wherein
said small RNA fragments are administered at a dose of at least 20 mg/week and for a time sufficient to raise the platelet count.

The invention further provides a preparation of small RNA fragments for use in the treatment of a solid tumour in a human patient, said small RNA fragments are bacterial small RNA fragments composed of single chain polyribonucleotides having 10 to 80 ribonucleotide units, and an overall ratio of purine bases to pyrimidine bases [(G+A)/(C+U)] of between 1.0 and 2.5, wherein said preparation is for simultaneous, separate or sequential use with at least one anti-cancer compound, and further wherein said small RNA fragments are administered at a dose of at least 60 mg to a subject at least every other day to maintain the platelet level or permit the recovery of platelets to a normal level during and after the administration of said anti-cancer compound.

The invention also provides a preparation of at least one anti-cancer compound for use in the treatment of a solid tumour in a human patient, wherein said preparation is for simultaneous, separate or sequential use with a preparation of small RNA fragments, which are bacterial small RNA fragments composed of single chain polyribonucleotides having 10 to 80 ribonucleotide units, and an overall ratio of purine bases to pyrimidine bases [(G+A)/(C+U)] of between 1.0 and 2.5, and further wherein said small RNA fragments are administered at a dose of at least 60 mg to a subject at least every other day to maintain the platelet level or permit the recovery of platelets to a normal level during and after the administration of said anti-cancer compound.

### BRIEF DESCRIPTION OF THE DRAWINGS

**Figure 1** illustrates the progressive failure of platelet recovery in a typical patient who did NOT receive the ReaLBuild® product as a component of their treatment.
**Figure 2** shows the results for a patient whose platelets are protected by the administration of the ReaLBuild® product.
**Figure 3** shows the protective effects of the ReaLBuild® product on the platelets in a patient who had undergone six prior chemotherapies.
**Figure 4** shows the results for a patient undergoing a chemotherapy regimen that included administration of the ReaLBuild® product.
**Figure 5** shows the results for a patient undergoing a chemotherapy regimen that included administration of the ReaLBuild® product.
**Figure 6** shows the results for a patient undergoing a chemotherapy regimen that included administration of the ReaLBuild® product.
**Figure 7** shows a summary of the results for all patients in the clinical trial (described in Example 1) correlating dose of the ReaLBuild® product with the effects on nadirs and recovery levels of platelets.
**Figure 8** shows the results for three patients with ITP before and after administration of the ReaLBuild® product.

### DETAILED DESCRIPTION OF THE INVENTION

The present invention provides therapeutic preparations based on administration of small RNA fragments, which may be used to prevent or ameliorate thrombocytopenia.

By "thrombocytopenia" is meant a condition characterized by defects in platelet production and processing, and includes both thrombocytopenia induced by anti-cancer drugs and congenital thrombocytopenia such as ITP.

By "treating" or "treatment" is meant to prevent or reduce the risk of occurrences of a disease or condition, or inhibit or ameliorate the symptoms of a disease or conditions, or accelerate the recovery from a disease or condition.

Chemotherapies are known to often cause extensive bone marrow suppression and thrombocytopenia. The present invention provides a novel chemotherapeutic regimen that incorporates administration of small RNA fragments before, during and/or after the administration of anti-cancer drugs with the result that bone marrow suppression is controlled, the therapy can be completed at full strength, and the life of the patient is extended. The success of this regimen was proven in a human clinical trial with cancer patients undergoing chemotherapies that normally cause extensive bone marrow suppression and thrombocytopenia. All of the patients completed the full course of therapy without any dose reduction of the drugs. The regimen was found to be effective in preventing thrombocytopenia even in patients who had undergone multiple previous chemotherapy treatments and sustained bone marrow damage. The present inventors have demonstrated for the first time that the administration of small RNA fragments has a broad application in chemotherapy and can effectively control and/or prevent bone marrow suppression and thrombocytopenia in patients suffering a range of cancers and undergoing therapy with a variety of anti-cancer drugs or combinations of drugs.

Additionally, the present inventors have demonstrated that the administration of small RNA fragments successfully increased the platelet counts in patients suffering from ITP.

Without wishing to be bound by any particular theory, the small RNA fragments are believed to function as primers for DNA synthesis in stem cells in bone marrow. By this action, the small RNA fragments stimulate the proliferation of the various white blood cell lineages thereby maintaining and/or restoring normal levels of lymphocytes and platelets (thrombocytes). Alternatively or additionally, it is possible that RNAs taken up by stem cells are degraded by RNAases, and the resulting ribonucleosides are converted to deoxyribonucleosides by ribonucleotide reductase thereby enriching the cells' resources for repair of genomic DNA damaged by anti-cancer drugs. An analysis of the localization of the RNA fragments following administration to animals revealed that the fragments concentrate rapidly in the bone marrow, but also in the liver, the adrenal gland, and several other organs. The localization in bone marrow is consistent with the notion that the RNA fragments stimulate the proliferation of the various white blood cell lineages. Moreover, the localization of the RNAs in the adrenals suggests that the RNAs protect and/or stimulate these glands, which may result in the release of hormones (e.g., adrenalin) that enhance energy and that may also stimulate the immune system. Such a stimulus would account for the boost in vitality experienced by many of the patients in the clinical trial.

Based on the discoveries of the present invention, the present invention provides therapeutic treatments that prevent or ameliorate thrombocytopenia based on administration of small RNA fragments.

In one embodiment, the present invention provides a preparation of small RNA fragments and at least one anti-cancer compound for use in the treatment of a solid tumour in a human patient, wherein
said small RNA fragments are bacterial small RNA fragments composed of single chain polyribonucleotides having 10 to 80 ribonucleotide units, and an overall ratio of purine bases to pyrimidine bases [(G+A)/(C+U)] of between 1.0 and 2.5, and further wherein
said small RNA fragments are administered at a dose of at least 60 mg to a subject at least every other day to maintain the platelet level or permit the recovery of platelets to a normal level during and after the administration of said anti-cancer compound.

In one embodiment, said small RNA fragments are for administration before, simultaneously with, or after the administration of said anti-cancer compound to said subject.

In one embodiment, the administration of said small RNA fragments to said subject is to begin the day before a cycle of treatment with said anti-cancer compound, and to continue either daily or every other day throughout the cycle of treatment with said anti-cancer compound, and for at least one additional week after completion of the cycle of treatment with said anti-cancer compound.

In one embodiment, the administration of said small RNA fragments to said subject is to begin the day before the first cycle of treatment with said anti-cancer compound.

In one embodiment, said subject has undergone at least one cycle of chemotherapy with an anti-cancer compound before the administration of said preparation of small RNA fragments.

In one embodiment, the preparation is for use in the treatment of a solid tumour selected from breast, esophagus, nasopharynx, colon, pancreas, cecum, lung, and prostate cancer.

In one embodiment, said anti-cancer compound or a combination of compounds comprising said compound causes bone marrow suppression.

In one embodiment, said compound is selected from those listed in Table 1 or Table 2.

In another embodiment, the present invention provides the use of small RNA fragments in the manufacture of a preparation for:
stimulating the proliferation and accelerating the regeneration of platelets in a cancer patient with a solid tumour undergoing chemotherapy with an anti-cancer drug; or
stimulating the proliferation and accelerating the regeneration of thrombocytes in a cancer patient with a solid tumour undergoing chemotherapy with an anti-cancer drug or;
stimulating the proliferation of white blood cells in a cancer patient with a solid tumour undergoing chemotherapy with an anti-cancer drug, wherein
said small RNA fragments are bacterial small RNA fragments composed of single chain polyribonucleotides having 10 to 80 ribonucleotide units, and an overall ratio of purine bases to pyrimidine bases [(G+A)/(C+U)] of between 1.0 and 2.5, and further wherein
said small RNA fragments are administered at a dose of at least 60 mg to a subject at least every other day to maintain the platelet level or permit the recovery of platelets to a normal level during and after the administration of said anti-cancer compound.

Platelets, also known as thrombocytes, are cell fragments that circulate in the blood and enable the formation of blood clots.

By "white blood cells" is meant to include basophils, eosinophils, neutrophils, mast cells, macrophages and lymphocytes (including T and B lymphocytes and NK cells).

In still another embodiment, the present invention a preparation of small RNA fragments for use in the treatment of thrombocytopenia in a human patient suffering from Immune Thrombocytopenic Pupura, wherein
said small RNA fragments are bacterial small RNA fragments composed of single chain polyribonucleotides having 10 to 80 ribonucleotide units, and an overall ratio of purine bases to pyrimidine bases [(G+A)/(C+U)] of between 1.0 and 2.5, and further wherein
said small RNA fragments are administered at a dose of at least 20 mg/week and for a time sufficient to raise the platelet count.

According to the present invention, a preparation of RNA fragments suitable for administration is composed of single stranded polyribonucleotides having 10 to 80, preferably 20-80, ribonucleotide units, and having an overall ratio of purine bases to pyrimidine bases [(G+A)/(C+U)] of between 1.0 and 2.5.

The preparation of RNA fragments can be made by degradation of the ribonucleic acids extracted from microorganisms, such as yeasts and bacteria, or from animal organs. An example of a bacterial strain suitable for use in extracting r-RNA is the non-pathogenic strain of *E*. *coli,* T3000 (K12), which belongs to the species that are normally hosts of the intestinal flora. The agents used for degradation include ribonucleases, such as a mammalian pancreatic ribonuclease or a ribonuclease from *Neurospora crassa*; as well as chemical reagents such as an alkali metal base (e.g., sodium hydroxide or potassium hydroxide), preferably at a final concentration of 0.1N in the reaction solution. Methods for preparing small RNA fragments have been described in e.g., U.S. Patent 4,335,239.

A suitable preparation of RNA fragments can be combined with one or more pharmaceutically acceptable carriers for administration. As used herein, a pharmaceutically acceptable carrier includes any and all solvents, dispersion media, isotonic agents and the like. Except insofar as any conventional media, agent, diluent or carrier is detrimental to the recipient or to the therapeutic effectiveness of the RNA fragments or the anti-cancer drug, its use in practicing the methods of the present invention is appropriate. The carrier can be liquid, semi-solid, e.g. pastes, or solid carriers. Examples of carriers include water, saline solutions, alcohol, sugar, gel, oils, lipids, liposomes, resins, porous matrices, binders, fillers, coatings, preservatives and the like, or combinations thereof. In accordance with the present invention, the active ingredients of the present pharmaceutical compositions can be combined with the carrier in any convenient and practical manner, e.g., by admixture, solution, suspension, emulsification, encapsulation, absorption and the like, and can be made in formulations such as tablets, capsules, powder, syrup, suspensions that are suitable for injections, implantations, inhalations, ingestions or the like.

Alternatively, preparations of RNA fragments or formulations containing a preparation of RNA fragments combined with one or more pharmaceutically acceptable carriers, which are suitable for administration, can be obtained commercially. A preferred commercial preparation of RNA fragments for use in the present regimen is the product marketed under the trade name "ReaLBuild®". A chemotherapeutic regimen that includes administration of the ReaLBuild® product is also referred to herein as either a ReaLBuild® regimen or more generally an RNA regimen.

The ReaLBuild® product is composed of a preparation of single-stranded chains of 10 to 80 ribonucleotides with the overall ratio of purine bases to pyrimidine bases (G+A)/(C+U) ranging from 1 to 2.5. This product is manufactured by extracting RNA from the *E*. *coli* strain K 12 using the methods described in U.S. Patent 4,335,239. The product is in a lyophilized powder form containing mannitol, which is added to give a sweet taste.

According to the present invention, a suitable preparation of RNA fragments can be administered to the patient via various routes, including the sublingual, oral, parenteral (e.g., intravenous, intraperitoneal, intradermal, subcutaneous or intramuscular) route. In a preferred embodiment, a suitable preparation of RNA fragments is given to the patient sublingually.

A suitable preparation of RNA fragments can be administered to the patient before, during, and/or after the administration of anti-cancer drugs. In a preferred embodiment, a suitable preparation of RNA fragments is given to the patient starting the day before the first cycle of a chemotherapy treatment and continuing either daily or every other day throughout the all cycles of treatment and for at least one additional week after completion of the last cycle.

Generally speaking, the daily dose of the RNA fragments is at least 20 milligrams, and up to 500 milligrams. The precise dose of the RNA fragments can be determined by the treating physician, taking into consideration of the patient's platelet level, the route of administration and other physical parameters such as age, weight and overall well being. In specific embodiments, the patient is given a daily dose sublingually of at least 40 mg, or at least 60 mg, or at least 80 mg.

As described hereinabove, a suitable preparation of RNA fragments can be incorporated in chemotherapies for treating a range of cancers, particularly solid tumors, including but are not limited to breast, esophagus, nasopharynx, colon, pancreas, cecum, lung, and prostate cancer.

Furthermore, the present chemotherapeutic regimen is applicable to any chemotherapeutic anti-cancer drug or a combination of drugs, especially those that cause bone marrow suppression and thrombocytopenia. Drugs that are not normally associated with bone marrow suppression or thrombocytopenia can also be included in the regimen. Examples of chemotherapeutic drugs and combinations of drugs for treating various cancers are listed below and are by no means limiting the scope of the present invention. Functional derivatives of a drug, i.e., derivatives that maintains the desired pharmacological effect of the drug, can also be used in practicing the present invention, such as salts, esters, amides, prodrugs, active metabolites, analogs and the like. The exact combinations, doses, timing and route of the administration of the chemotherapeutic drugs can be determined by the treating oncologist using standard procedures (e.g., by considering Body Surface Area calculations).

**Table 1**

| **Chemotherapy (Brand)** |
|---|
| Avastin |
| Capecitabane (Xeloda) |
| Carboplatin (Paraplatin) |
| Cetuximab (Erbitux) |
| Cisplatin (CDDP) |
| Cisplatin (Platinol) |
| Cyclophosphamide (Cytoxan) |
| Docetaxel (Taxotere) |
| Doxorubicin (Adriamycin) |
| Etoposide (VePesid) |
| Femara |
| Floxuridine (FUDR) |
| Gemcitibine |
| Ifosfamide (Ifex) |
| Iressa |
| Irinotecan (Camptosar) |
| Leucovorin (Immunex) |
| *Mesna (Mesnex)* |
| Mini Ice |
| Mitomycin (Mutamycin) |
| Navelbine |
| Oxaliplatin (Eloxatin) |
| Paclitaxel (Abraxane) |
| Paclitaxel (Taxol) |
| Pemetrexed (Alimta) |
| Tarceva |
| Temozolomide |
| Topotecan (Hycamtin) |
| Trastuzumab (Herceptin) |
| Zolemata (Zoledronic Acid) |

**Table 2**

| **Chemotherapy Combinations** |
|---|
| Leucovorin/Floxuridine/Cisplatin/Mitomycin |
| Pemetrexed/Cisplatin/Cetuximab |
| Docetaxel/Carboplatin |
| Docetaxel/Carboplatin |
| Pemetrexed/Cisplatin |
| Leucovorin/Floxuridine |
| Doxorubicin |
| Mini ICE (Mesna, Ifosfamide, Carbo, Etopo) |
| Doxorubicin |
| Paclitaxel |
| Cetuximab/Irinotecan |
| Pemetrexed/Cisplatin |
| Docetaxel/Carboplatin |
| Paclitaxel/Carbolplatin |
| Leucovorin/Floxuridine/Topotecan |
| Leucovorin/Floxuridine/Topotecan |
| Leucovorin/Floxuridine/CetuximablMitomycin |
| Leucovorin/Floxuridine/Cisplatin/Mitomycin |
| Leucovorin/Floxuridine/Cisplatin/Mitomycin |
| Leucovorin/Floxuridine/Oxaliplatin |
| Docetaxel/Carboplatin/Pemetrexed/Cisplatin |
| Docetaxel/Carboplatin |
| Cetuximab/Irinotecan |
| Doxorubicin/Cyclophosphamide |
| Docetaxel/Carboplatin |
| Pemetrexed |
| Capecitabane |
| Capecitabane/Oxaliplatin/Irinotecan |
| Docetaxel |
| Etoposide/Trastuzumab/Zoledronic Acid/Carboplatin/Mesna/Ifosfamide |
| Leucovorin/Floxuridine/Cisplatin/Mitomycin |

Other drugs that are beneficial to a patient undergoing chemotherapy can also be included in the regimen. For example, the drugs Neupogen® or Neulasta®can be used to help support the levels of other white blood cell populations (e.g. neutrophils). Such additional beneficial drugs can be administered simultaneously with a preparation of RNA fragments, or with the chemotherapeutic drug or drugs; or alternatively, can be administered separately.

The present invention is further illustrated and by no means limited by the following examples.

### Example 1. Chemotherapeutic Regimens Incorporating Administration Of The ReaLBuild® Product Prevented Thrombocytopenia In Advanced Cancer Patients

The ReaLBuild® Product was tested in a Phase I Clinical Trial at a major cancer treatment center (Cancer Treatment Centers of America), designed both to examine the safety and the efficacy of the ReaLBuild® product. The participants in the trial suffered from a range of advanced cancers (including breast, esophagus, nasopharynx, colon, pancreas), and many had either been extensively pretreated having failed multiple previous chemotherapies or were suffering from metastatic disease. The patients were administered a variety of chemotherapeutic drugs, including drugs well known to induce bone marrow suppression and thrombocytopenia. Blood cell counts, including platelet counts were monitored as they customarily are for patients undergoing aggressive chemotherapies. Under the circumstances, this trial represents a severe test for any agent intended to promote white blood cell production and prevent thrombocytopenia.

In this trial eligibility for receiving RealBuild® RNA fragments was contingent on potential onset of thrombocytopenia, defined as platelet counts less than 80,000 platelets/ml or having demonstrated a chemotherapy induced thrombocytopenia of 80,000 or less in their most recent cycle. The patients began taking the RealBuild® product as soon as this trigger was attained and continued taking the product every other day for the remaining cycles of treatment.

**Table 3** provides a summary of the results of the clinical trial with the ReaLBuild® product. The first six columns from left to right list A) the patient ID numbers; B) the sex of the patients; C) number of prior chemotherapy treatments; D) the type of cancer; E) the Body Surface Area calculation used to determine the dose of the chemotherapeutic drugs; and F) the drug regimen the patient was administered. The column 'RNA Dose' (G) refers to the initial dose of RNA in milligrams that the patient started on. 'Nadir prior RNA' (H) lists the lowest recorded platelet counts the patient had before starting treatment with RNA. 'Recovery post RNA' (I) refers to the platelet count induced by the RNA. 'Nadir post RNA' (J) lists the lowest platelet counts after a further cycle of chemotherapy. 'Recovery post RNA' (K) lists the platelets count following recovery from the treatment cycle. The 'Final Dose RNA' column (L) lists the dose of RNA taken at the completion of treatment and serves as an indicator of dose escalation(s). Note that patient 26 never entered therapy and so was never administered RNA.

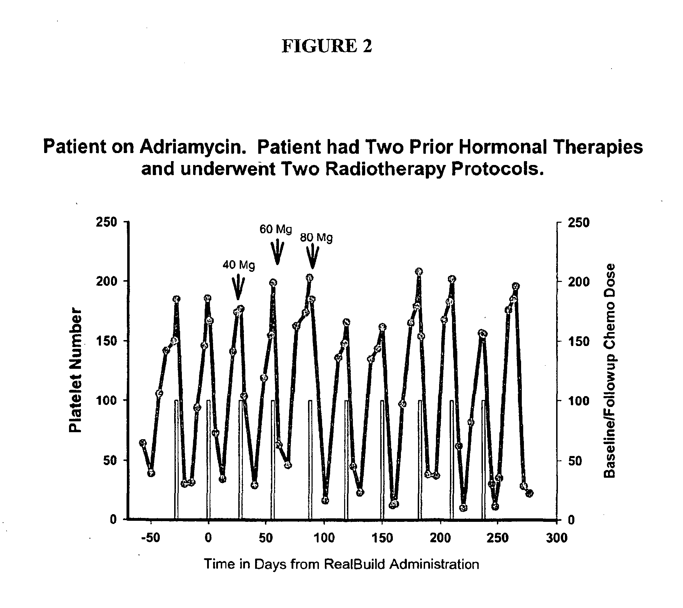

Typical results showing the platelet counts of four patients in the trial are shown in **Figure 2** through **Figure 6****.** In all the figures, the number of platelets (in red, y axis) are plotted against the time the patient has been in chemotherapy (x-axis). The vertical bars represent chemotherapy doses. In all cases, the number of platelets oscillated: the platelet counts fell after administration of the anti-cancer drugs and then rose as the patients bone marrow recovered from the effects of the treatment. Another treatment induced a drop in platelets, followed again by a recovery in the counts and so on.

**Figure 1** illustrates the progressive failure of platelet recovery in a typical patient who did not receive the ReaLBuild® product as a component of their treatment. This serves as a negative control for the trial. For this control patient, the low nadirs and the falling platelet counts associated with each recovery forced two dose reductions in chemotherapy: the shorter vertical lines signify smaller doses than the protocol specified. Finally, after 175 days, the platelets did not come back and the therapy was terminated.

**Figure 2** shows the results for a patient whose platelets are protected by the ReaLBuild® product. The drop in platelets induced by each round of chemotherapy was followed by rapid recovery of the platelets counts - well into the normal range. There was no dose reduction or suspension of the treatment and the patient completed the prescribed therapy without suffering the effects of thrombocytopenia.

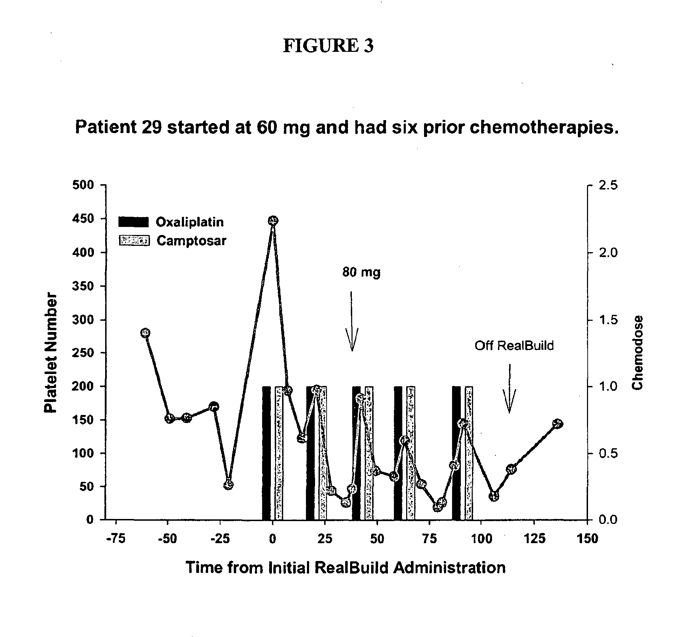

The graph in **Figure 3** also shows the protective effects of the ReaLBuild® product on the platelets for a patient who had undergone six prior chemotherapies. The peak numbers of platelets were not as high as those for the patient represented in **Figure 2****,** but the fact is that the ReaLBuild® product kept the recovery counts in the normal range (>100,000 platelets per ml) and the full course of therapy was completed without dose reduction or any other complication.

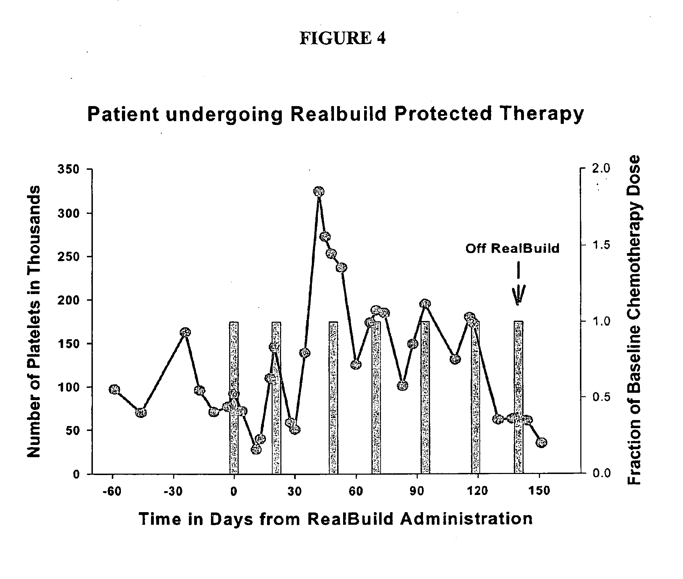

**Figure 4** shows the results for a patient undergoing a regimen based on administration of the ReaLBuild® product. This patient had four previous chemotherapies in addition to two courses of radiation treatment. Again, the regimen maintained the patient's platelet levels within the normal range and the therapy was completed without dose reduction.

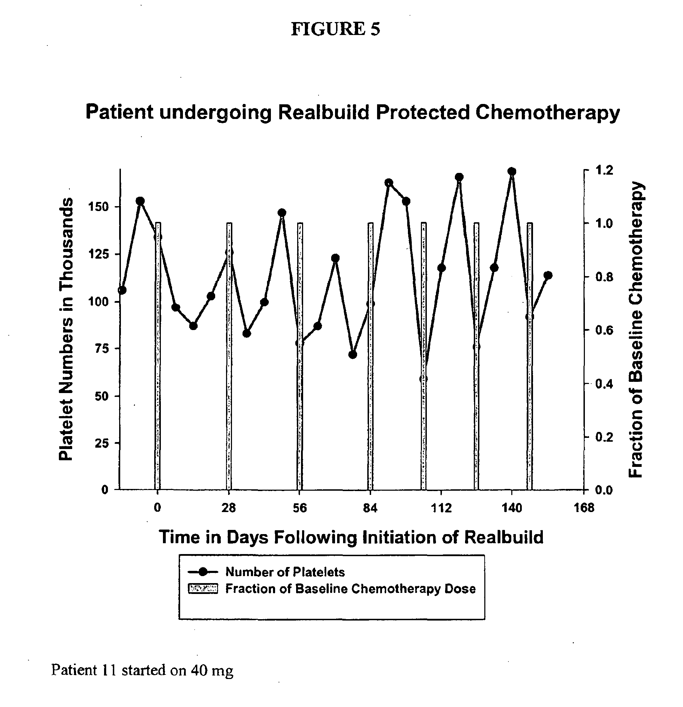

The effectiveness of a regimen based on administration of the ReaLBuild® product in protecting platelets levels is clearly demonstrated by comparing the data for the patient in **Figure 5** with the control shown in **Figure 1****.** The regimen in this case not only kept the recovery platelet levels in the normal range, but also kept the nadirs relatively high. This effect was seen in a number of patients, indicating that this chemotherapy regimen enables complete treatments in which platelet counts remain well above critical levels.

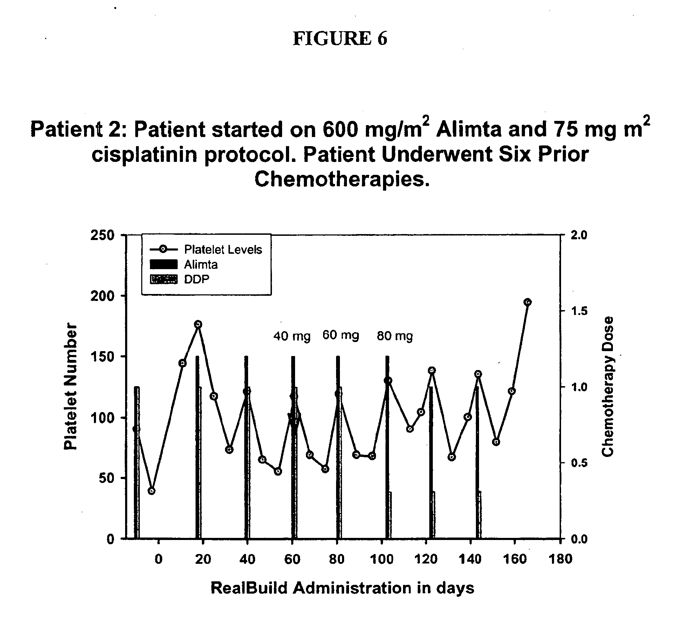

**Figure 6** shows the results for a patient undergoing a regimen based on administration of the ReaLBuild® product. This patient had six previous chemotherapies. Even in this case, the regimen effectively supported the recovery of platelet levels into the normal range.

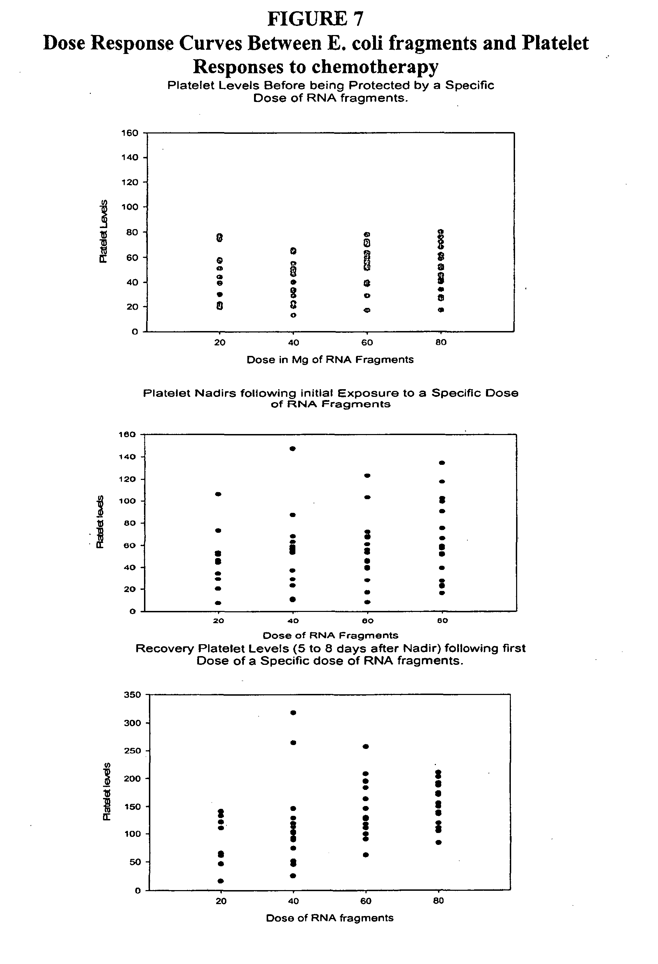

The success of the chemotherapeutic regimen based on administration of the ReaLBuild® product is summarized in **Figure 7****.** This figure shows the effect of the regimen on the platelet nadirs and the platelet recoveries for each patient in the trial and for each dose of the ReaLBuild® product. The graph at the top shows the patients' platelet levels prior to protection by administration of the ReaLBuild® product. The graph in the middle shows the effect of specific doses of the ReaLBuild® product on platelet nadirs. The overall improvements were noted at the 80 mg dose. The graph at the bottom shows the platelet recovery levels following the first dose of each specific dose of the ReaLBuild® product. The scale in this graph is different than the one for the pre-regimen baseline counts shown in the graph at the top. Platelet recoveries are significantly improved especially at the 60 and 80 mg. doses.

### Example 2. The Potential Of The ReaLBuild® Product: Transforming Cancer From A Terminal Disease To A Chronic Disease

The clinical trial discussed in the previous section also revealed several important aspects of using a regimen based on the administration of the ReaLBuild® product in chemotherapy patients.

First, the ReaLBuild® product is not associated with any negative side effects and there is absolutely no toxicity. In fact, many of the patients in the trial reported that they felt better while taking the product and others commented that they had more energy. These comments indicate a positive effect on quality of life.

Second, the protective effect on platelet counts in the trial participants was strictly associated with administration of the ReaLBuild® product. Patients who were removed from the trial because the therapy failed and their tumors progressed also failed to maintain platelet levels when they went off the ReaLBuild® product. At the completion of the trial, all of the patients were removed from the ReaLBuild® product and many of them were either slow to recover their platelets or failed to do so.

Third, the product does not super-induce the numbers of white blood cells. Patients in the trial did not overproduce platelets-rather these cells returned to normal range. This aspect suggests a regulatory balance that is not overridden by the ReaLBuild® product. Indeed, the dose escalations in the trial provided some insight into what might be the best dose for an individual patient, but no adverse effects occurred from the lowest to the highest dose. The ReaLBuild® product is tolerated with ease, even among this very sick population.

Fourth, the ReaLBuild® product does not interfere with chemotherapy in any way and is specific in its stimulation of white blood cells; it does not stimulate the growth of tumor cells. This means that the regimen can be assembled from a wide selection of anti-cancer drugs whenever there is a risk of bone marrow suppression and thrombocytopenia.

Finally, for all of the 31 patients on trial, there were no dose reductions and no fatalities. It appears that the ReaLBuild® product extended the lives of the patients.

These observations indicate that the regimens of the present invention open a new horizon in cancer therapy. In a broader sense, a regimen of the present invention can be applied not only with late stage patients undergoing chemotherapy as in the clinical trial, but also in patients with newly diagnosed and early stage cancers. The regimen frees oncologists from the restrictions imposed by bone marrow suppression and thrombocytopenia and enables them to develop and optimize chemotherapies with maximal anti-cancer effect. The regimen is a significant factor in the transformation of cancer from a terminal to a chronic disease and it will continue to be useful with newly developed drugs that provide a basis for chemotherapies that enable complete cancer cures.

**TABLE 3**

| PT ID | SEX | # prior the | Site | BSA (m2) | Chemotherapy Drugs | RNA Dose | Nadir prior RNA | Recovery post RNA | Nadir post RNA | Recovery post RNA | Final Dose RNA |
|---|---|---|---|---|---|---|---|---|---|---|---|
| 1 | F | 4 | Colon | 1.80 | Leucovorin/Floxuridine/Cisplatin/Mitomycin | 20 | 19 | 188.0 | 28 | 150 | 20 |
| 2 | F | 6 | Breast | 1.72 | Pemetrexed/Osplatin/Cetuximab | 20 | 39 | 90.0 | 73 | 121 | 80 |
| 3 | F | 1 | Breast | 2.02 | Docetaxel/Carboplatin | 20 | 77 | 234.0 | 52 | 336 | 20 |
| 4 | | 2 | Nasopharyn | 2.16 | Docetaxel/Carboplatin | 20 | 57 | 162.0 | 46 | 196 | 40 |
| 5 | F | 8 | Breast | 1.77 | Pemetrexed/Cisplatin | 20 | 32 | 128.0 | 20 | 65 | 40 |
| 6 | F | 2 | Colon | 1.82 | Leucovorin/Floxuridine | 20 | 51 | 161.0 | 44 | 133 | 20 |
| 7 | F | 4 | unknown on | 1.76 | Doxorubicin | 20 | 21 | 121.0 | 51 | 131 | 40 |
| 8 | F | 6 | Breast | 2.08 | Minl ICE (Mesna, uosramuie; carbo, Etopo) | 20 | 44 | 169.0 | 29 | 95 | 40 |
| 9 | F | 3 | Breast | 1.62 | Doxorubicin | 20 | 30 | 186.0 | 34 | 177 | 80 |
| 10 | F | 5 | Breast | 2.01 | Paclitaxel | 20 | 58 | 248.0 | 106 | 222 | 20 |
| 11 | M | 4 | Anal /Rectal | 2.10 | Cetuximab/Irinotecan | 40 | 66 | 153.0 | 87 | 126 | 80 |
| 12 | F | 3 | Breast | 1.56 | Pemetrexed/Cisplatin | 40 | 13 | 248.0 | 59 | 178 | 80 |
| 13 | M | 0 | Esophagus | 1.84 | Docetaxel/Carboplatin | 40 | 55 | 384.0 | 20 | | 40 |
| 14 | F | 1 | Pancreas | 1.86 | Paclitaxel/Carbolplatin | 40 | 49 | 154.0 | | | 40 |
| 15 | F | 4 | unknown on | 1.78 | leucovortn/F1oxurtdlne/Topotecan | 40 | 33 | 594.0 | 147 | 413 | 80 |
| 16 | F | 1 | Pancreas | 1.78 | Leucovorin/Floxuridine/Topotecan | 40 | 66 | 244.0 | 87 | 289 | 40 |
| 17 | F | 2 | Pancreas | 1.61 | Leucovorin/Fluxuridine/Cetuximab/Mitomicin | 40 | 65 | 173.0 | 53 | 152 | 80 |
| 18 | M | 1 | Pancreas | 2.14 | Leucovorin/Floxuridine/Cisplatin/Mitomycin | 40 | 23 | 409.0 | 63 | 251 | 80 |
| 19 | M | 0 | Pancreas | 1.95 | Leucovorin/Floxuridine/Cisplatin/Mitomycin | 40 | 64 | 257.0 | 23 | 172 | 80 |
| 20 | M | 0 | Colon | 2.03 | Leucovorin/Floxuridine/Oxaliplatin | 40 | 40 | 131.0 | 56 | 147 | 80 |
| 21 | M | 1 | | 1.74 | Docetaxel/Carboplatin/Pemetrexed/Cisplatin | 60 | 60 | 140.0 | 45 | 129 | 80 |
| 22 | M | 0 | Breast | 1.71 | Docetaxel/Carboplatin | 60 | 56 | 226.0 | 226 | 360 | 80 |
| 23 | F | 3 | Breast | 1.74 | Cetuximab/Irinotecan | 60 | 51 | 356.0 | 39 | 339 | 80 |
| 24 | F | 0 | Lung | 1.73 | Doxorubicin/Cyclophosphamide | 60 | 64 | 190.0 | 42 | 142 | 80 |
| 25 | F | 1 | Breast | 1.84 | Docetaxel/Carboplatin | 60 | 73 | 132,0 | 67 | 173 | 60 |
| 26 | | | | | | 60 | | | | | |
| 27 | F | 4 | Breast | 1.52 | Pemetrexed | 60 | 71 | 92.0 | 28 | 146 | 80 |
| 28 | F | 4 | Colon | 1.77 | Capecitabane | 60 | 51 | 59.0 | 72 | 109 | 60 |
| 29 | F | 3 | Lung | 1.65 | Capecitabane/Oxaliplatin/Irinotecan | 60 | 52 | 447.0 | 123 | 195 | 80 |
| 30 | F | 3 | Breast | 1.58 | Docetaxel | 60 | 70 | 168.0 | 72 | 183 | 60 |
| 31 | F | 2 | Lung | 1.55 | Etoposide/Trastuzumab/Zoledronic Acid/Carbop | 80 | 62 | 428.0 | 22 | 433 | 80 |
| 32 | M | 1 | Colon | 2.25 | Leucovorin/Floxuridine/Cisplatin/Mitomycin | 80 | 34 | 382.0 | 27 | 284 | 80 |

### Example 3. The ReaLBuild® Product is an Effective Treatment for Immune Thrombocytopenic Purpura.

Although the cause of critically low platelet counts in patients with Immune Thrombocytopenic Purpura is the presence of anti-platelet antibodies rather than chemotherapeutic drugs, the RealBuild® product effectively induces platelet proliferation in cases of ITP just as it does in cancer patients with drug-induced thrombocytopenia.

Patient information, doses of the ReaLBuild® product, and platelet counts before and after treatment for three individuals diagnosed with Immune Thrombocytopenic Purpura are shown in **Table 4.** Patient R.S. is a male with severe ITP as indicated by pretreatment platelet counts between 49,000 and 31,000. Administration of the ReaLBuild® product significantly increased the platelet counts of this patient (107,000). Patients V.A. and D.JY. both suffer from ITP and their platelet counts before treatment were below normal. Administration of the ReaLBuild® product restored normal levels of platelets in both of these patients.

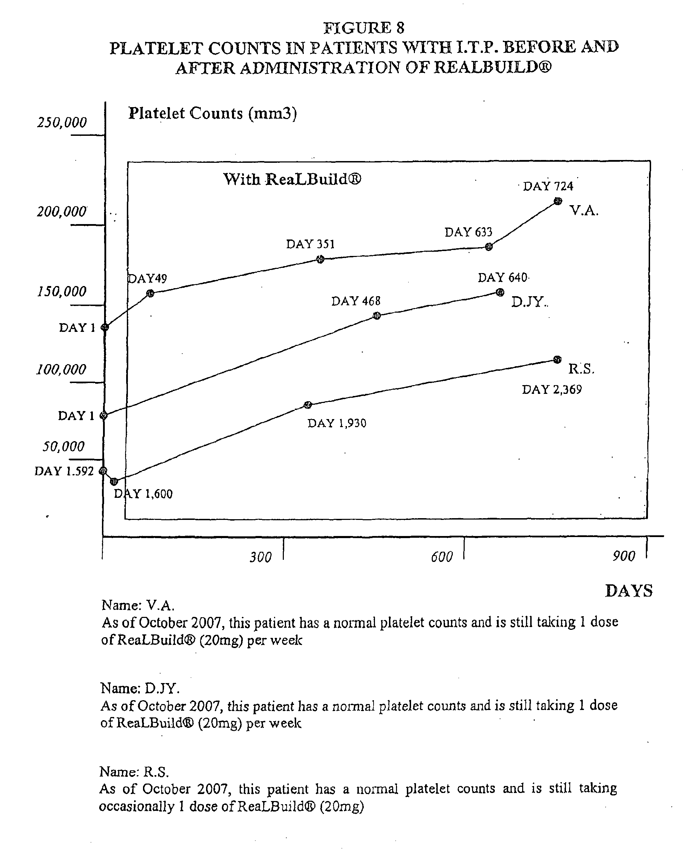

**Figure 8** presents the data from **Table 4** in graphical form. The curves for each patient demonstrate the increases in platelet counts associated with administration of the ReaLBuild® product. It should be emphasized that these patients suffered from chronic ITP and had a history of low platelet counts. The improvement in their platelet counts was directly correlated with administration of the ReaLBuild® product and they were not taking any other medications that influence white blood cell populations during the time periods indicated in **Table 4** and **Figure 8****.**

**TABLE 4**

| | | | | PLATELET COUNTS mm3 | | | |
|---|---|---|---|---|---|---|---|
| | DIAGNOSIS: | Dose of ReaLBuild(20mg) | Without RLB | Without RLB | Without RLB | With RLB | With RLB |
| Name: R.S. | Severe Immune | the first mortth 2 per week then 1 every 10 days | Day 1 | Day 1,592 | Day 1,600 | Day 1,930 | Day 2,369 |
| SEX: F | Trombocytopenic | | | | | | |
| Weight: 109 lbs | Purpura | | | | | | |
| | | | 49,000 | 39,000 | 31,000 | 87,000 | 107,000 |

| | | | | PLATELET COUNTS mm3 | | | |
|---|---|---|---|---|---|---|---|
| | DIAGNOSIS: | Dose of ReaLBuild(20mg) | Without RLB | With RLB | With RLB | With RLB | With RLB |
| Name: V.A. | Immune | 1 dose per week | Day 1 | Day 49 | Day 351 | Day 633 | Day 724 |
| SEX: M | Trombocytopenic | | | | | | |
| Weight: 187 lbs | Purpura | | | | | | |
| | | | 141,000 | 151,000 | 172,000 | 177,000 | 209,000 |

| | | | PLATELET COUNTS mm3 | | | | |
|---|---|---|---|---|---|---|---|
| | DIAGNOSIS: | Dose of ReaLBuild(20mg) | Without RLB | with RLB | with RLB | | |
| Name: D.JY | Immune | 1 dose per week dose | Day 1 | Day 468 | Day 640 | | |
| SEX: M | Trombocytopenic | | | | | | |
| Weight: 143 lbs | Purpura | | | | | | |
| | | | 74,900 | 134,000 | 147,000 | | |

## Claims

1. A preparation of small RNA fragments and at least one anti-cancer compound for use in the treatment of a solid tumour in a human patient, wherein
said small RNA fragments are bacterial small RNA fragments composed of single chain polyribonucleotides having 10 to 80 ribonucleotide units, and an overall ratio of purine bases to pyrimidine bases [(G+A)/(C+U)] of between 1.0 and 2.5, and further wherein
said small RNA fragments are administered at a dose of at least 60 mg to a subject at least every other day to maintain the platelet level or permit the recovery of platelets to a normal level during and after the administration of said anti-cancer compound.

2. A preparation for use according to claim 1, wherein
said small RNA fragments are for administration before, simultaneously with, or after the administration of said anti-cancer compound to said subject.

3. A preparation for use according to claim 2, wherein the administration of said small RNA fragments to said subject is to begin the day before a cycle of treatment with said anti-cancer compound, and to continue either daily or every other day throughout the cycle of treatment with said anti-cancer compound, and for at least one additional week after completion of the cycle of treatment with said anti-cancer compound.

4. A preparation for use according to claim 3, wherein the administration of said small RNA fragments to said subject is to begin the day before the first cycle of treatment with said anti-cancer compound.

5. A preparation for use according to claim 1, wherein said subject has undergone at least one cycle of chemotherapy with an anti-cancer compound before the administration of said preparation of small RNA fragments.

6. A preparation for use according to claim 1, wherein said solid tumor is selected from the group consisting of breast, esophagus, nasopharynx, colon, pancreas, cecum, lung, and prostate cancer.

7. A preparation for use according to claim 1, wherein said anti-cancer compound or a combination of compounds comprising said compound causes bone marrow suppression.

8. The preparation for use according to claim 7, wherein said compound is selected from those listed in Table 1 or Table 2.

9. Use of small RNA fragments in the manufacture of a preparation for:
stimulating the proliferation and accelerating the regeneration of platelets in a cancer patient with a solid tumour undergoing chemotherapy with an anti-cancer drug; or
stimulating the proliferation and accelerating the regeneration of thrombocytes in a cancer patient with a solid tumour undergoing chemotherapy with an anti-cancer drug or;
stimulating the proliferation of white blood cells in a cancer patient with a solid tumour undergoing chemotherapy with an anti-cancer drug, wherein
said small RNA fragments are bacterial small RNA fragments composed of single chain polyribonucleotides having 10 to 80 ribonucleotide units, and an overall ratio of purine bases to pyrimidine bases [(G+A)/(C+U)] of between 1.0 and 2.5, and further wherein
said small RNA fragments are administered at a dose of at least 60 mg to a subject at least every other day to maintain the platelet level or permit the recovery of platelets to a normal level during and after the administration of said anti-cancer compound.

10. A preparation of small RNA fragments for use in the treatment of thrombocytopenia in a human patient suffering from Immune Thrombocytopenic Pupura, wherein
said small RNA fragments are bacterial small RNA fragments composed of single chain polyribonucleotides having 10 to 80 ribonucleotide units, and an overall ratio of purine bases to pyrimidine bases [(G+A)/(C+U)] of between 1.0 and 2.5, and further wherein
said small RNA fragments are administered at a dose of at least 20 mg/week and for a time sufficient to raise the platelet count.

11. Use of a preparation of small RNA fragments and at least one anti-cancer compound for the manufacture of a medicament for the treatment of a solid tumour in a human patient, wherein
said small RNA fragments are bacterial small RNA fragments composed of single chain polyribonucleotides having 10 to 80 ribonucleotide units, and an overall ratio of purine bases to pyrimidine bases [(G+A)/(C+U)] of between 1.0 and 2.5, and further wherein
said small RNA fragments are administered at a dose of at least 60 mg to a subject at least every other day to maintain the platelet level or permit the recovery of platelets to a normal level during and after the administration of said anti-cancer compound.

12. Use of a preparation of small RNA fragments for the manufacture of a medicament for the treatment of thrombocytopenia in a human patient suffering from Immune Thrombocytopenic Pupura wherein
said small RNA fragments are bacterial small RNA fragments composed of single chain polyribonucleotides having 10 to 80 ribonucleotide units, and an overall ratio of purine bases to pyrimidine bases [(G+A)/(C+U)] of between 1.0 and 2.5, and further wherein
said small RNA fragments are administered at a dose of at least 20 mg/week and for a time sufficient to raise the platelet count.

13. A preparation of small RNA fragments for use in the treatment of a solid tumour in a human patient, said small RNA fragments are bacterial small RNA fragments composed of single chain polyribonucleotides having 10 to 80 ribonucleotide units, and an overall ratio of purine bases to pyrimidine bases [(G+A)/(C+U)] of between 1.0 and 2.5, wherein
the treatment comprises the simultaneous, separate or sequential use of said preparation with a preparation of at least one anti-cancer compound, and further wherein said small RNA fragments are administered at a dose of at least 60 mg to a subject at least every other day to maintain the platelet level or permit the recovery of platelets to a normal level during and after the administration of said anti-cancer compound.

14. A preparation of at least one anti-cancer compound for use in the treatment of a solid tumour in a human patient, wherein the treatment comprises the simultaneous, separate or sequential use of said preparation with a preparation of small RNA fragments, which are bacterial small RNA fragments composed of single chain polyribonucleotides having 10 to 80 ribonucleotide units, and an overall ratio of purine bases to pyrimidine bases [(G+A)/(C+U)] of between 1.0 and 2.5, and further wherein said small RNA fragments are administered at a dose of at least 60 mg to a subject at least every other day to maintain the platelet level or permit the recovery of platelets to a normal level during and after the administration of said anti-cancer compound.

## Patentansprüche

1. Präparat kleiner RNA-Fragmente und wenigstens eine Anti-Krebs-Verbindung zur Verwendung in der Behandlung eines soliden Tumors bei einem menschlichen Patienten, wobei
die kleinen RNA-Fragmente bakterielle kleine RNA-Fragmente sind, die aus Einzelketten-Polyribonucleotiden mit 10 bis 80 Ribonucleotideinheiten und mit einem Gesamtverhältnis von Purinbasen zu Pyrimidinbasen [(G+A)/(C+U)] von zwischen 1,0 und 2,5 bestehen, und wobei außerdem
die kleinen RNA-Fragmente mit einer Dosis von wenigstens 60 mg an das Subjekt wenigstens jeden zweiten Tag während und nach der Verabreichung der Anti-Krebs-Verbindung verabreicht werden, um den Thrombozytenspiegel aufrecht zu erhalten oder die Wiederherstellung von Thrombozyten zu einem normalen Spiegel zu erlauben.

2. Präparat zur Verwendung nach Anspruch 1, wobei die kleinen RNA-Fragmente zur Verabreichung vor, gleichzeitig mit oder nach der Verabreichung der Anti-Krebs-Verbindung an das Subjekt bestimmt sind.

3. Präparat zur Verwendung nach Anspruch 2, wobei die Verabreichung der kleinen RNA-Fragmente an das Subjekt einen Tag vor einem Behandlungszyklus mit der Anti-Krebs-Verbindung beginnen soll und entweder täglich oder jeden zweiten Tag durch den Behandlungszyklus mit der Anti-Krebs-Verbindung und für wenigstens eine zusätzlich Woche nach Beendigung des Behandlungszyklus mit der Anti-Krebs-Verbindung fortgesetzt werden soll.

4. Präparat zur Verwendung nach Anspruch 3, wobei die Verabreichung der kleinen RNA-Fragmente an das Subjekt am Tag vor dem ersten Behandlungszyklus mit der Anti-Krebs-Verbindung beginnen soll.

5. Präparat zur Verwendung nach Anspruch 1, wobei das Subjekt wenigstens einen Chemotherapie-Zyklus mit einer Anti-Krebs-Verbindung vor der Verabreichung des Präparats kleiner RNA-Fragmente durchgemacht hat.

6. Präparat zur Verwendung nach Anspruch 1, wobei der solide Tumor aus der Gruppe, bestehend aus Brust-, Ösophagus-, Nasopharynx-, Colon-, Pankreas-, Blinddarm-, Lungen- und Prostatakrebs, ausgewählt ist.

7. Präparat zur Verwendung nach Anspruch 1, wobei die Anti-Krebs-Verbindung oder eine Kombination von Verbindungen, die die Verbindung umfasst, eine Knochenmarkssuppression verursacht.

8. Präparat zur Verwendung nach Anspruch 7, wobei die Verbindung aus denen, die in Tabelle 1 oder Tabelle 2 aufgelistet sind, ausgewählt ist.

9. Verwendung kleiner RNA-Fragmente zur Herstellung eines Präparates zum:
Stimulieren der Proliferation und Beschleunigen der Regeneration von Thrombozyten bei einem Krebspatienten mit einem soliden Tumor, der sich einer Chemotherapie mit einem Anti-Krebs-Arzneimittel unterzieht, oder
Stimulieren der Proliferation und Beschleunigen der Regeneration von Thrombozyten bei einem Krebspatienten mit einem soliden Tumor, der sich einer Chemotherapie mit einem Anti-Krebs-Arzneimittel unterzieht, oder
Stimulieren der Leukozyten-Proliferation bei einem Krebspatienten mit einem soliden Tumor, der sich einer Chemotherapie mit einem Anti-Krebs-Arzneimittel unterzieht, wobei
die kleinen RNA-Fragmente bakterielle kleine RNA-Fragmente sind, die aus Einzelketten-Polyribonucleotiden mit 10 bis 80 Ribonucleotideinheiten und mit einem Gesamtverhältnis von Purinbasen zu Pyrimidinbasen [(G+A)/(C+U)] von zwischen 1,0 und 2,5 bestehen, und wobei außerdem
die kleinen RNA-Fragmente mit einer Dosis von wenigstens 60 mg an das Subjekt wenigstens jeden zweiten Tag während und nach der Verabreichung der Anti-Krebs-Verbindung verabreicht werden, um den Thrombozytenspiegel aufrecht zu erhalten oder die Wiederherstellung von Thrombozyten zu einem normalen Spiegel zu erlauben.

10. Präparat kleiner RNA-Fragmente zur Verwendung bei der Behandlung von Thrombozytopenie bei einem menschlichen Patienten, der an Immuner Thrombozytopenischer Pupura leidet, wobei
die kleinen RNA-Fragmente bakterielle kleine RNA-Fragmente sind, die aus Einzelketten-Polyribonucleotiden mit 10 bis 80 Ribonucleotideinheiten und mit einem Gesamtverhältnis von Purinbasen zu Pyrimidinbasen [(G+A)/(C+U)] von zwischen 1,0 und 2,5 bestehen und wobei außerdem
die kleinen RNA-Fragmente mit einer Dosis von wenigstens 20 mg/Woche und für eine Zeit, die ausreichend ist, um die Thrombozytenzahl zu erhöhen, verabreicht werden.

11. Verwendung eines Präparats kleiner RNA-Fragmente und wenigstens einer Anti-Krebs-Verbindung zur Herstellung eines Medikaments für die Behandlung eines soliden Tumors bei einem menschlichen Patienten, wobei
die kleinen RNA-Fragmente bakterielle kleine RNA-Fragmente sind, die aus Einzelketten-Polyribonucleotiden mit 10 bis 80 Ribonucleotideinheiten und mit einem Gesamtverhältnis von Purinbasen zu Pyrimidinbasen [(G+A)/(C+U)] von zwischen 1,0 und 2,5 bestehen, und wobei außerdem
die kleinen RNA-Fragmente mit einer Dosis von wenigstens 60 mg an das Subjekt wenigstens jeden zweiten Tag während und nach der Verabreichung der Anti-Krebs-Verbindung verabreicht werden, um den Thrombozytenspiegel aufrecht zu erhalten oder die Wiederherstellung von Thrombozyten zu einem normalen Spiegel zu erlauben.

12. Verwendung eines Präparates kleiner RNA-Fragmente für die Herstellung eines Medikaments für die Behandlung von Thrombozytopenie bei einem menschlichen Patienten, der an Immuner Thrombozytopenischer Pupura leidet, wobei
die kleinen RNA-Fragmente bakterielle kleine RNA-Fragmente sind, die aus Einzelketten-Polyribonucleotiden mit 10 bis 80 Ribonucleotideinheiten und mit einem Gesamtverhältnis von Purinbasen zu Pyrimidinbasen [(G+A)/(C+U)] von zwischen 1,0 und 2,5 bestehen, und wobei außerdem
die kleinen RNA-Fragmente mit einer Dosis von wenigstens 20 mg/Woche und für eine Zeit, die ausreichend ist, um die Leukozytenzahl zu erhöhen, verabreicht werden.

13. Präparat kleiner RNA-Fragmente zur Verwendung bei der Behandlung eines soliden Tumors bei einem menschlichen Patienten, wobei die kleinen RNA-Fragmente bakterielle kleine RNA-Fragmente sind, die aus Einzelketten-Polyribonucleotiden mit 10 bis 80 Ribonucleotideinheiten und mit einem Gesamtverhältnis von Purinbasen zu Pyrimidinbasen [(G+A)/(C+U)] von zwischen 1,0 und 2,5 bestehen, wobei die Behandlung die gleichzeitige, getrennte oder sequentielle Verwendung des Präparates mit einem Präparat wenigstens einer Anti-Krebs-Verbindung umfasst und wobei außerdem die kleinen RNA-Fragmente mit einer Dosis von wenigstens 60 mg an einen Patienten wenigstens jeden zweiten Tag während und nach der Verabreichung der Anti-Krebs-Verbindung verabreicht werden, um den Thrombozytenspiegel aufrecht zu erhalten oder die Wiederherstellung von Thrombozyten zu einem normalen Spiegel zu erlauben.

14. Präparat wenigstens einer Anti-Krebs-Verbindung zur Verwendung bei der Behandlung eines soliden Tumors bei einem menschlichen Patienten, wobei die Behandlung die gleichzeitige, getrennte oder sequentielle Verwendung des Präparats mit einem Präparat kleiner RNA-Fragmente, die bakterielle kleine RNA-Fragmente sind, die aus Einzelketten-Polyribonucleotiden mit 10 bis 80 Ribonucleotideinheiten und mit einem Gesamtverhältnis von Purinbasen zu Pyrimidinbasen [(G+A)/(C+U)] von zwischen 1,0 und 2,5 bestehen, umfasst und wobei außerdem die kleinen RNA-Fragmente mit einer Dosis von wenigstens 60 mg an einen Patienten wenigstens jeden zweiten Tag während und nach der Verabreichung der Anti-Krebs-Verbindung verabreicht werden, um den Thrombozytenspiegel aufrecht zu erhalten oder die Wiederherstellung von Thrombozyten zu einem normalen Spiegel zu erlauben.

## Revendications

1. Préparation de petits fragments d'ARN et d'au moins un composé anticancéreux, pour utilisation dans le traitement d'une tumeur solide chez un patient humain, dans laquelle :
- lesdits petits fragments d'ARN sont de petits fragments d'ARN bactérien composés de polyribonucléotides simple-brin comportant de 10 à 80 ribonucléotides et présentant un rapport global des bases puriques aux bases pyrimidiques, soit (G+A)/(C+U), de 1,0 à 2,5 ;
et étant en outre entendu que :
- lesdits petits fragments d'ARN sont administrés en une dose d'au moins 60 mg à un sujet, au moins tous les deux jours, afin de maintenir le taux de plaquettes ou de permettre la restauration d'un taux normal de plaquettes, pendant et après l'administration dudit composé anticancéreux.

2. Préparation pour utilisation, conforme à la revendication 1, étant entendu que lesdits petits fragments d'ARN sont destinés à être administrés avant, pendant ou après l'administration dudit composé anticancéreux audit sujet.

3. Préparation pour utilisation, conforme à la revendication 2, étant entendu que l'administration desdits petits fragments d'ARN audit sujet doit débuter la veille du démarrage d'un cycle de traitement avec ledit composé anticancéreux et se poursuivre chaque jour ou tous les deux jours pendant tout le cycle de traitement avec ledit composé anticancéreux, et au moins une semaine supplémentaire après l'achèvement du cycle de traitement avec ledit composé anticancéreux.

4. Préparation pour utilisation, conforme à la revendication 3, étant entendu que l'administration desdits petits fragments d'ARN audit sujet doit débuter la veille du démarrage du premier cycle de traitement avec ledit composé anticancéreux.

5. Préparation pour utilisation, conforme à la revendication 1, étant entendu que ledit sujet a subi au moins un cycle de chimiothérapie avec un composé anticancéreux avant l'administration de ladite préparation de petits fragments d'ARN.

6. Préparation pour utilisation, conforme à la revendication 1, étant entendu que ladite tumeur solide est choisie dans l'ensemble formé par les cancers du sein, de l'oesophage, du naso-pharynx, du côlon, du pancréas, du cæcum, du poumon, et de la prostate.

7. Préparation pour utilisation, conforme à la revendication 1, étant entendu que ledit composé anticancéreux, ou une combinaison de composés comprenant ledit composé, provoque une dépression de la moelle osseuse.

8. Préparation pour utilisation, conforme à la revendication 7, étant entendu que ledit composé est choisi parmi ceux qui figurent dans le Tableau 1 ou le Tableau 2.

9. Utilisation de petits fragments d'ARN dans la fabrication d'une préparation conçue :
- pour stimuler la prolifération des plaquettes et accélérer leur régénération chez un patient cancéreux présentant une tumeur solide et subissant une chimiothérapie avec un médicament anticancéreux,
- ou pour stimuler la prolifération des thrombocytes et accélérer leur régénération chez un patient cancéreux présentant une tumeur solide et subissant une chimiothérapie avec un médicament anticancéreux,
- ou pour stimuler la prolifération des leucocytes et accélérer leur régénération chez un patient cancéreux présentant une tumeur solide et subissant une chimiothérapie avec un médicament anticancéreux ;
étant entendu que
- lesdits petits fragments d'ARN sont de petits fragments d'ARN bactérien composés de polyribonucléotides simple-brin comportant de 10 à 80 ribonucléotides et présentant un rapport global des bases puriques aux bases pyrimidiques, soit (G+A)/(C+U), de 1,0 à 2,5 ;
et étant en outre entendu que :
- lesdits petits fragments d'ARN sont administrés en une dose d'au moins 60 mg à un sujet, au moins tous les deux jours, afin de maintenir le taux de plaquettes ou de permettre la restauration d'un taux normal de plaquettes, pendant et après l'administration dudit composé anticancéreux.

10. Préparation de petits fragments d'ARN, pour utilisation dans le traitement de la thrombocytopénie chez un patient humain souffrant d'un purpura thrombopénique immunologique, dans laquelle
- lesdits petits fragments d'ARN sont de petits fragments d'ARN bactérien composés de polyribonucléotides simple-brin comportant de 10 à 80 ribonucléotides et présentant un rapport global des bases puriques aux bases pyrimidiques, soit (G+A)/(C+U), de 1,0 à 2,5 ;
et étant en outre entendu que :
- lesdits petits fragments d'ARN sont administrés en une dose d'au moins 20 mg par semaine et pendant suffisamment longtemps pour que la numération plaquettaire s'élève.

11. Utilisation d'une préparation de petits fragments d'ARN et d'au moins un composé anticancéreux, pour la fabrication d'un médicament conçu pour le traitement d'une tumeur solide chez un patient humain, dans laquelle préparation
- lesdits petits fragments d'ARN sont de petits fragments d'ARN bactérien composés de polyribonucléotides simple-brin comportant de 10 à 80 ribonucléotides et présentant un rapport global des bases puriques aux bases pyrimidiques, soit (G+A)/(C+U), de 1,0 à 2,5 ;
et étant en outre entendu que :
- lesdits petits fragments d'ARN sont administrés en une dose d'au moins 60 mg à un sujet, au moins tous les deux jours, afin de maintenir le taux de plaquettes ou de permettre la restauration d'un taux normal de plaquettes, pendant et après l'administration dudit composé anticancéreux.

12. Utilisation d'une préparation de petits fragments d'ARN, pour la fabrication d'un médicament conçu pour le traitement de la thrombocytopénie chez un patient humain souffrant d'un purpura thrombopénique immunologique, dans laquelle préparation
- lesdits petits fragments d'ARN sont de petits fragments d'ARN bactérien composés de polyribonucléotides simple-brin comportant de 10 à 80 ribonucléotides et présentant un rapport global des bases puriques aux bases pyrimidiques, soit (G+A)/(C+U), de 1,0 à 2,5 ;
et étant en outre entendu que :
- lesdits petits fragments d'ARN sont administrés en une dose d'au moins 20 mg par semaine et pendant suffisamment longtemps pour que la numération plaquettaire s'élève.

13. Préparation de petits fragments d'ARN, pour utilisation dans le traitement d'une tumeur solide chez un patient humain, dans laquelle lesdits petits fragments d'ARN sont de petits fragments d'ARN bactérien composés de polyribonucléotides simple-brin comportant de 10 à 80 ribonucléotides et présentant un rapport global des bases puriques aux bases pyrimidiques, soit (G+A)/(C+U), de 1,0 à 2,5, étant entendu que le traitement comporte le fait d'utiliser simultanément, séparément ou successivement ladite préparation et une préparation d'au moins un composé anticancéreux, et étant en outre entendu que lesdits petits fragments d'ARN sont administrés en une dose d'au moins 60 mg à un sujet, au moins tous les deux jours, afin de maintenir le taux de plaquettes ou de permettre la restauration d'un taux normal de plaquettes, pendant et après l'administration dudit composé anticancéreux.

14. Préparation d'au moins un composé anticancéreux pour utilisation dans le traitement d'une tumeur solide chez un patient humain, étant entendu que le traitement comporte le fait d'utiliser simultanément, séparément ou successivement ladite préparation et une préparation de petits fragments d'ARN, lesquels sont de petits fragments d'ARN bactérien composés de polyribonucléotides simple-brin comportant de 10 à 80 ribonucléotides et présentant un rapport global des bases puriques aux bases pyrimidiques, soit (G+A)/(C+U), de 1,0 à 2,5, et étant en outre entendu que lesdits petits fragments d'ARN sont administrés en une dose d'au moins 60 mg à un sujet, au moins tous les deux jours, afin de maintenir le taux de plaquettes ou de permettre la restauration d'un taux normal de plaquettes, pendant et après l'administration dudit composé anticancéreux.
